(19) Europäisches Patentamt
European Patent Office
Office européen des brevets

(11) Veröffentlichungsnummer: **0 046 565**
**B1**

(12) **EUROPÄISCHE PATENTSCHRIFT**

(45) Veröffentlichungstag der Patentschrift: **13.04.83**

(51) Int. Cl.³: **A 61 K 31/435,** C 07 D 519/00 // (C07D519/00, 471/00, 471/00)

(21) Anmeldenummer: **81106402.1**

(22) Anmeldetag: **18.08.81**

(54) **Neues Sparteinderivat, Verfahren zu seiner Herstellung, das Derivat enthaltende Arzneimittel und Verfahren zur Herstellung der Arzneimittel.**

(30) Priorität: **27.08.80 DE 3032219**

(43) Veröffentlichungstag der Anmeldung: **03.03.82 Patentblatt 82/9**

(45) Bekanntmachung des Hinweises auf die Patenterteilung: **13.04.83 Patentblatt 83/15**

(84) Benannte Vertragsstaaten: **AT BE CH DE FR GB IT LI LU NL SE**

(56) Entgegenhaltungen:
**TETRAHEDRON, Band 29, 1973, Seiten 2195—2202 Dublin, IE. D. HERLEM et al.: »Reactions photochimiques d'amines tertiaires et d'alcaloides-III« * Seiten 2197, 2201 ***
**CHEMICAL ABSTRACTS, Band 77, 1972, Seite 238, Nr. 58768n Columbus, Ohio, U.S.A. V.I. VINOGRADOVA et al.: »Dithermamine, a new bimolecular alkaloid from Termopsis lanceolata«**

(73) Patentinhaber: **Kali-Chemie Pharma GmbH, Hans-Böckler-Allee 20 Postfach 220, D-3000 Hannover 1 (DE)**

(72) Erfinder: **Hachmeister, Bernd, Dr.rer.nat.Dipl.-Chem., Möwenkamp 4, D-3004 Isernhagen 1 (DE)**
Erfinder: **Kehrbach, Wolfgang, Dr.rer.nat.Dipl.-Chem., Altenbekener Damm 41, D-3000 Hannover (DE)**
Erfinder: **Kühl, Ulrich, Dr.med.vet., Franz-Burger-Strasse 10, D-3007 Gehrden (DE)**
Erfinder: **Buschmann, Gerd, Dr.med.vet., Matthäikirchstrasse 27, D-3000 Hannover 81 (DE)**

(74) Vertreter: **Lauer, Dieter, Dr., c/o Kali-Chemie Aktiengesellschaft Postfach 220, D-3000 Hannover 1 (DE)**

EP 0 046 565 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neues Sparteinderivat, Verfahren zu seiner Herstellung, das Derivat enthaltende Arzneimittel und Verfahren zur Herstellung der Arzneimittel

Die Erfindung betrifft 17S,17'S-Bisspartein, Verfahren zu seiner Herstellung sowie das Derivat enthaltende Arzneimittel und Verfahren zur Herstellung solcher Arzneimittel.

Es ist bekannt, daß sich Spartein und 17-Alkylderivate des Sparteins durch wertvolle pharmakologische, insbesondere antiarrhythmische Wirkungen auszeichenen (DE-OS 2 360 475, Europäische Patentanmeldung 79 103 315.2).

Es ist vorteilhaft, wenn ein Antiarrhythmikum eine große therapeutische Breite aufweist, d. h., das Verhältnis aus toxischer Dosis zur Wirkdosis muß groß sein. Weiterhin ist es vorteilhaft, wenn ein Antiarrhythmikum auch nach per-oraler Applikation über einen längeren Zeitraum wirkt.

Die bekannten Sparteinderivate haben den Nachteil, daß die antiarrhythmische Wirkung nach per-oraler Applikation nur gering oder die Wirkung nur kurz ist.

Aufgabe der vorliegenden Erfindung ist es, einen Wirkstoff mit Sparteingrundgerüst mit günstigerem Wirkungsprofil als die der bekannten Sparteinderivate und dieses Derivat enthaltende Arzneimittel bereitzustellen.

Die Lösung dieser Aufgabe wird durch die Bereitstellung von 17S,17'S-Bisspartein erfüllt.

Verschiedene dimere Sparteine sind in der Literatur beschrieben: (H.-G. Boit, »Ergebnisse der Alkaloid-Chemie bis 1960« S. 197, Akademie-Verlag Berlin, 1961; R. H. F. Manske, »The Alkaloids«, Vol IX, S. 194, Academic Press, New York 1967).

Bei diesen $\alpha,\beta,\gamma$ bzw. $\delta$-Diplospartyrinen handelt es sich um Sparteinderivate mit chemischen Bindungen zwischen den Kohlenstoffatomen 17 und 5' bzw. 12' bzw. 14', in keinem Fall aber um Verbindungen vom Typ des 17S,17'S-Bissparteins. Diese Verbindung ist neu.

Das erfindungsgemäße neue dimere Sparteinderivat kann hergestellt werden durch Umsetzung von 17-Hydroxyspartein oder 17-Dehydrosparteinsalz, vorzugsweise dem Perchlorat-Salz, mit einem Alkalimetall oder mit aktivem Magnesium in einem Lösungsmittel.

Die so erhaltene Verbindung kann gewünschtenfalls mit physiologisch verträglichen Säuren in seine Säureadditionssalze überführt werden.

Als Lösungsmittel kommen vorzugsweise aprotische Lösungsmittel in Frage. Bevorzugt werden Äther verwendet, insbesondere Tetrahydrofuran, Diäthyläther, Ethylenglycoldimethyläther und/oder deren Gemische.

Vorteilhafterweise führt man die Umsetzung bei der Siedetemperatur des Lösungsmittels aus, bei höher siedenden Lösungsmitteln kann die Umsetzung auch unterhalb der Siedetemperatur durchgeführt werden.

Magnesium mit aktiver Oberfläche kann nach verschiedenen Verfahren gewonnen werden:

a) Durch Amalgamierung mit einem Quecksilbersalz, z. B. durch Umsetzung von metallischem Magnesium mit Quecksilber(II)-chlorid.
b) Durch Umsetzung mit einer katalytischen Menge von organischen Monohalogenverbindungen, beispielsweise mit Bromveratrol und Methyljodid.
c) Durch Ausfällung von fein verteiltem Magnesium aus einem wasserfreien Magnesiumsalz mit einem unedleren Metall wie z. B. Natrium oder Kalium.
d) Durch Behandlung des Magnesiums mit einem Protonenspender. In einer besonderen Variante des Verfahrens wird als Protonenspender eine Säure verwendet.

Bei dieser Aktivierungsmethode wird das Magnesium in Gegenwart des als Ausgangsverbindung eingesetzten Sparteins, insbesondere des 17-Hydroxysparteins aktiviert. Obwohl die Dimerisierung zum Bisspartein auch in einem protischen Lösungsmittel in Gegenwart eines Protonenspenders möglich ist — z. B. im System Wasser/Halogenwasserstoff — ist es von Vorteil, als Lösungsmittel ein Gemisch aus Protonenspender und aprotischem Lösungsmittel zu verwenden. Der Anteil des Protonenspenders am Gesamtvolumen der flüssigen Phase beträgt dabei 0,5 bis 50 Vol.-%, vorzugsweise 1 bis 30 Vol.-%. Setzt man als Protonenspender eine Säure ein, so wird eine organische Säure, insbesondere Essigsäure oder Trifluoressigsäure bevorzugt.

Erfolgt die Umsetzung mit einem Alkalimetall, so wird vorzugsweise fein verteiltes Alkalimetall, insbesondere fein verteiltes Natrium eingesetzt.

Die Isolierung des basischen 17S,17'S-Bissparteins aus dem Reaktionsgemisch und seine Reinigung erfolgen auf an sich bekannte Weise. Insbesondere kann es aus alkalischem Medium extrahiert werden und das Rohprodukt durch Chromatographie und/oder Kristallisation gereinigt werden.

Die erfindungsgemäß erhältlichen Säureadditionssalze erhält man durch Umsetzung des basischen 17S,17'S-Bissparteins mit Säuren, die physiologisch unbedenkliche Salze bilden. Als Beispiele solcher Salze seien genannt Halogenide, insbesondere Chloride, Fumarate, Naphthalinsulfonate, Sulfate, Phosphate, Citrate, Maleinate, Tartrate.

Die nach dem erfindungsgemäßen Verfahren hergestellten Salze sind sowohl in wäßriger Lösung als auch in fester Form stabil und haltbar und können durch Vermischen mit pharmazeutisch üblichen Träger- und/oder Hilfsstoffen zu Arzneimitteln verarbeitet werden.

Das erfindungsgemäße 17S,17'S-Bisspartein weist günstigere pharmakologische, insbesondere antiarrhythmische Eigenschaften auf als Spartein und die bekannten 17-Alkylsparteine. So zeigt das neue dimere Spartein schon bei wesentlich geringerer Dosis eine vergleichbare Beeinflussung der funktionellen Refraktärzeit bei geringerer bzw. vergleichbarer Toxizität. Weiterhin zeigt die erfindungsgemäße Verbindung bei oraler Gabe selbst nach 3 Stunden noch eine gute antiarrhythmische Wirkung. Hingegen konnte bei den bekannten Sparteinderivaten unter gleichen Bedingungen keine Wirkung mehr nachgewiesen werden.

Die Überlegenheit des erfindungsgemäßen Wirkstoffs ergibt sich aus dem in Tabelle 1 angeführten Vergleich mit Spartein und einem repräsentativen Vertreter aus der 17-Alkylsparteinreihe.

In der Tabelle ist im einzelnen aufgeführt:

Die akute Toxizität bei per-oraler (p. o.) Applikation an männlichen NMRI-Mäusen der Gewichtsklasse 18 bis 22 g. Als $LD_{50}$ ist dabei diejenige Dosis in µmol/kg definiert, bei der die Mortalitätsrate am 7. Tag nach Applikation 50% der Versuchstiere beträgt. Die $LD_{50}$ wurde durch Probitanalyse [L. Cavalli-Sforza, Grundbegriffe der Biometrie, insbesondere der statistischen Methoden bei der Wertbemessung biologisch wirksamer Substanzen, Gustav Fischer Verlag, Stuttgart (1964)] berechnet.

Der Einfluß der Wirksubstanz auf mit Aconitin infundierten männlichen Wistar-Ratten der Gewichtsklasse 280 bis 350 g gemäß der Methode nach Raschak [M. Raschak, Arzneim.-Forsch. (Drug Res.) 25 (4) (1975), 639–641].

Angegeben ist die wirksame Dosis in µmol/kg, bei welcher die Zeiten bis zum Auftreten von durch das Aconitin ausgelösten Arrhythmien signifikant gegenüber einer Kontrollgruppe, welche nur das Vehikel erhalten hatte, verzögert wurde. Bei der wirksamen Dosis mußten die Zeiten bis zum Auftreten von Arrhythmien signifikant verlängert werden, und zwar mußte die Substanz gegen mindestens zwei der nach Aconitininfusion auftretenden typischen Arrhythmieformen (Extrasystolen, ventrikuläre Tachykardie, Kammerflattern) wirksam sein. Die in 2%iger Tylose®MH 50 suspendierte Prüfsubstanz wurde den Tieren 3 Stunden vor Beginn der Aconitin-Infusion peroral appliziert.

Als Standarddosis wurde 1/10 des peroralen $LD_{50}$-Wertes an der Maus verabreicht. Erwies sich eine Prüfsubstanz als bei dieser Dosis wirksam, so wurde die Dosis reduziert. Als Signifikanztest wurde der t-Test nach Student nach Logarithmierung der Effektwerte verwendet (Lothar Sachs, Statistische Auswertungsmethoden, Springer Verlag, Berlin, Heidelberg, New York, 1969).

Die Verlängerung der funktionellen Refraktärzeit (fRZ) am isolierten linken Vorhof von weiblichen Albino-Pirbright-white-Meerschweinchen der Gewichtsklasse 300 bis 400 g gemäß der Doppelreizmethode von Govier [W. C. Govier, J. Pharmakol. Exp. Ther. 148 (1) (1965), 100–105]. Angegeben ist diejenige Konzentration in µmol/l, bei der es 18 Minuten nach Applikation zu einer Verlängerung der funktionellen Refraktärzeit auf 125% (EK 125%) kommt.

Da die Daten in der Dimension µmol/kg bzw. µmol/l angegeben sind, ist in der Tabelle außerdem noch das errechnete Molekulargewicht (MG) der Wirksubstanz angegeben.

Tabelle 1

| Wirksubstanz | MG [g/mol] | $LD_{50}$ µmol/kg p. o. | Aconitin-Test wirksame Dosis µmol/kg p. o. | fRZ EK 125% µmol/l |
|---|---|---|---|---|
| Sparteinsulfat | 423 | 540 | > 54 | 34 |
| 17-Pentylsparteintartrat | 695 | 1800 | >180 | 9 |
| 17S,17'S-Bisspartein × 2 Weinsäure L (+) | 767 | >1900 | 33 | 5 |

Folgende Beispiele sollen den Gegenstand der Erfindung näher erläutern:

Das 17-Hydroxyspartein wurde gemäß DE-OS 2 825 117.4 aus Spartein gewonnen.

Das 17-Dehydrosparteinperchlorat wurde nach M. Rink und K. Grabowski, Arch. Pharm. 289 (1956) 695 aus 17-Hydroxyspartein hergestellt.

## Beispiel 1

### Dimerisierung von 17-Dehydrospartein-Perchlorat mit frisch ausgefälltem, fein verteiltem Magnesium unter Verwendung von Kalium

4 g wasserfreies Magnesiumchlorid werden zusammen mit 1,5 g Kalium 3,5 Stunden in 100 ml absolutem Tetrahydrofuran unter Rückfluß erhitzt. Dabei wird das Magnesium als schwarze Dispersion abgeschieden. Zu der auf Raumtemperatur abgekühlten Suspension werden 12 g

17-Dehydrospartein-Perchlorat gegeben und das Ganze 2,5 Std. unter guten Rühren zum Sieden erhitzt. Anschließend gibt man, während der Reaktionsansatz weiter erhitzt wird, 10 ml Isopropanol hinzu, kühlt dann die Suspension ab und säuert mit verdünnter Salzsäure an. Nachdem das im Überschuß eingesetzte Magnesium in Lösung gegangen ist, wird zweimal mit je 100 ml Methylenchlorid extrahiert. Die wäßrige Phase wird nach Zugabe von 20 g Ammoniumchlorid mit 20%iger Natronlauge alkalisch eingestellt und anschließend zweimal mit je 100 ml Diäthyläther extrahiert. Die organische Phase wird im Wasserstrahlvakuum eingedampft und der Rückstand mit Methylenchlorid aufgenommen. Nach Trocknung über Magnesiumsulfat wird das Lösungsmittel im Vakuum abgezogen.

Nach säulenchromatographischer Reinigung über Aluminiumoxid, Akt.-Stufe II-III mit Hexan/Diäthyläther 90 : 10 werden 6,2 g reines, kristallines 17S,17'S-Bisspartein isoliert.

Ausbeute: 73,8%; Fp.: 178° C; $\alpha_D^{20}$: −101,4° in $CH_2Cl_2$.

Ein kristallines Ditartratsalz (Fp.: 186° C) erhält man durch Zusatz der berechneten Menge L(+)Weinsäure zu einer heißen Lösung der Bissparteinbase in Isopropanol.

In gleicher Weise kann das amorphe Tetrahydrochloridsalz gewonnen werden, wenn man zur Bissparteinbase in Isopropanol überschüssige äthanolische Salzsäure gibt und die Lösung zur Trockene eindampft.

## Beispiel 2

### Dimerisierung von 17-Hydroxyspartein mit frisch ausgefälltem, fein verteiltem Magnesium unter Verwendung von Kalium

Die Reaktion wird analog zu dem in Beispiel 1 beschriebenen Verfahren durchgeführt. Bei Zugabe von 5 g 17-Hydroxyspartein (anstatt der 12 g 17-Dehydrosparteinperchlorat) werden nach säulenchromatographischer Reinigung 2,4 g (51,4% Ausbeute) Bisspartein isoliert.

## Beispiel 3

### Dimerisierung von 17-Dehydrosparteinperchlorat mit frisch ausgefälltem, fein verteiltem Magnesium unter Verwendung von Natrium

Die Reaktion wird analog zu dem in Beispiel 1 beschriebenen Verfahren durchgeführt. Das Magnesium wird jedoch nicht mit Kalium, sondern mittels einer 30%igen Natriumdispersion in Toluol ausgefällt. Man erhält das dimere Produkt nach dem beschriebenen Aufarbeitungsverfahren in 49,5% Ausbeute.

## Beispiel 4

### Dimerisierung von 17-Hydroxyspartein mit amalgamiertem Magnesium

Nach 2stündiger Vorbehandlung von 2,4 g Magnesiumspänen mit 7 g Quecksilberchlorid in 150 ml siedendem Tetrahydrofuran wird eine Lösung von 10 g 17-Hydroxyspartein in 100 ml Tetrahydrofuran zugetropft. Nach weiteren 2,5 Stunden Rückflußkochen wird die Lösung in der in Beispiel 1 angegebenen Weise aufgearbeitet. Man erhält 4,6 g 17S,17'S-Bisspartein (–49,3% Ausbeute).

## Beispiel 5

### Dimerisierung von 17-Dehydrospartein-Perchlorat mit amalgamiertem Magnesium

Unter den Bedingungen des Beispiels 4 werden 10 g Dehydrospartein-Perchlorat mit amalgamiertem Magnesium (erhalten aus 1,45 g Magnesiumspäne und 3,25 g Quecksilberchlorid) in 75 ml Tetrahydrofuran umgesetzt.

Das nach der Säure-Base-Trennung erhaltene Rohprodukt (siehe Beispiel 1) wird ohne Säulenchromatographie durch Kristallisation gereinigt. Hierzu wird das Rohprodukt in heißem Dichlormethan gelöst. Nach Zugabe von Aceton fällt das 17S,17'S-Bisspartein aus.

Ausbeute: 6,14 g (=87,4%).

## Beispiel 6

### Dimerisierung von 17-Dehydrospartein-Perchlorat mit Magnesium unter Zusatz von organischem Monohalogenverbindungen

Zu 3,3 g Magnesiumspänen wird eine Lösung von 0,3 ml Methyljodid in 25 ml Diäthyläther getropft. Nach 10 min Rückflußsieden werden 29,3 g Bromveratrol in 25 ml Diäthyläther zugegeben. Der Reaktionsansatz wird noch 2 Stunden unter Rückfluß erhitzt, dann wird eine Suspension von 15 g 17-Dehydrospartein-Perchlorat in 30 ml Tetrahydrofuran zugegeben und weitere 4 Stunden gekocht. Nach der beschriebenen Aufarbeitung und Reinigung (Beispiel 1) werden 3,2 g (=30,5%) dimeres Produkt isoliert.

## Beispiel 7

### Dimerisierung von 17-Dehydrospartein-Perchlorat mit fein verteiltem Natrium

10 g 17-Dehydrospartein-Salz werden zusammen mit 1,7 g einer 40%igen Dispersion von Natrium in Paraffin (Teilchendurchmesser ~10 µ) in 300 ml Tetrahydrofuran 8 Stunden zum Sieden erhitzt. Nach Zugabe von 10 ml Isopropanol wird der Reaktionsansatz mit verdünnter Salzsäure hydrolysiert, das ausgefallene Paraffin abgesaugt und das wäßrige Filtrat analog zu dem in Beispiel 1 beschriebenen Verfahren weiter aufgearbeitet. Es werden 4,55 g (=65%) 17S,17'S-Bisspartein isoliert.

## Beispiel 8

### Dimerisierung von 17-Hydroxyspartein mit Magnesium/Protonen-Spender

7,8 g 17-Hydroxyspartein werden in einem Gemisch aus 7,8 ml Eisessig und 39 ml Tetrahydrofuran gelöst. Nach Zugabe von 0,76 g Magnesium erhitzt man dann 2 h unter Rückfluß.

Aufarbeitung:

Variante A:

Nach Einengen des Reaktionsansatzes wird mit 20%iger wäßriger Ammoniumchloridlösung aufgenommen, mit konzentrierter wäßriger Ammoniaklösung ein pH-Wert von 9 eingestellt und dreimal mit Methylenchlorid extrahiert.
Rohausbeute: 5,6 g (76%).

Variante B:

In das mit Tetrahydrofuran auf 100 ml verdünnte Reaktionsgemisch leitet man gasförmiges Ammoniak ein, so daß sich ein pH-Wert von 11 einstellt. Nach Zugabe von 240 ml Äther wird der Niederschlag abgesaugt und das getrocknete Filtrat eingedampft.
Rohausbeute: 6,2 g (85%).

Reinigung:

Chromatographie von 10,7 g Rohprodukt an 230 g Tonerde mit Cyclohexan als Laufmittel.
Ausbeute: 7,8 g (73% bezogen auf Rohprodukt).
Das 17S,17'S-Bisspartein kristallisiert aus einem Gemisch aus Methylenchlorid und Aceton, sowie aus Äthanol.

## Beispiel 9

### Dimerisierung von 17-Hydroxyspartein mit Magnesium/Protonen-Spender

2,1 g 17-Hydroxyspartein werden in einem Gemisch aus 1 ml Trifluoressigsäure und 30 ml Tetrahydrofuran gelöst. Nach Zugabe von 0,4 g Magnesium erhitzt man dann 3 Stunden unter Rückfluß.

Aufarbeitung nach Beispiel 8, Variante A.
Rohausbeute 1,5 g (76%).

## Beispiel 10

### Dimerisierung von 17-Hydroxyspartein mit Magnesium/Protonen-Spender

1,4 g 17-Hydroxyspartein werden in einem Gemisch aus 1,4 ml Essigsäure und 10 ml Äthylenglykoldimethyläther gelöst. Nach Zugabe von 0,13 g Magnesium erhitzt man dann 2 Stunden unter Rückfluß.

Aufarbeitung nach Beispiel 8, Variante A.
Rohausbeute: 1,0 g (76%).

Die Beispiele 11 – 14 beschreiben Verfahren zur Herstellung von Arzneimitteln, die erfindungsgemäße Wirkstoffe enthalten.

## Beispiel 11

### Tabletten

Zusammensetzung:

| | |
|---|---|
| 17S,17'S-Bissparteinditratrat | 20,0 mg |
| Lactose | 40,0 mg |
| Maisstärke | 30,0 mg |
| Kollidon®25 | 5,0 mg |
| Aerosil®200 | 0,2 mg |
| Stearinsäure | 3,0 mg |
| | 98,2 mg |

Herstellung:

17S,17'S-Bissparteinditartrat wird mit Lactose und Maisstärke im Diosna-Mischer 1 min vorgemischt. Man feuchtet mit einer wäßrigen Lösung von Kollidon®25 gründlich durch und passiert das noch feuchte Granulat durch ein 1,5 mm-Sieb. Nach dem Trocknen wird die Masse durch ein 1-mm-Sieb getrieben, mit Aerosil®200 und Stearinsäure vermischt und die so erhaltene preßfertige Mischung auf dem Rundläufer zu Tabletten verpreßt. Die Tabletten wiegen im Mittel 98,2 mg, so daß pro Tablette 20 mg 17S,17'S-Bissparteinditartrat enthalten sind.

## Beispiel 12

### Kapseln

Zusammensetzung:

| | |
|---|---|
| 17S,17'S-Bissparteinditartrat | 20,0 mg |
| Lactose sprühgetrocknet | 50,0 mg |
| Maisstärke | 25,0 mg |
| Aerosil®200 | 0,5 mg |
| Magnesiumstearat | 1,5 mg |
| | 97,0 mg |

Herstellung:

17S,17'S-Bissparteinditartrat wird zusammen mit Lactose und Maisstärke 20 min im Kubusmischer vermischt. Aerosil®200 und Magnesiumsterat werden, nachdem sie durch ein 0,2-mm-Sieb passiert wurden, mit dem Ansatz vereinigt und innerhalb von 5 min vermischt. Auf einer automatischen Kapselfüllmaschine wird die Pulvermischung in Kapseln der Größe 4 abgefüllt. Jede Kapsel enthält im Mittel 97 mg Pulvermischung, entsprechend 20 mg 17S,17'S-Bissparteinditartrat.

Beispiel 13

Ampullen

Zusammensetzung:

| | |
|---|---|
| 17S,17'S-Bissparteintetrachlorid | 5,0 mg |
| Natriumchlorid | 9,0 mg |
| Bidestilliertes Wasser, ad | 1,0 ml |

Herstellung:

17S,17'S-Bissparteintetrachlorid und Natriumchlorid werden in bidestilliertem Wasser gelöst. Die Lösung wird filtriert, in Ampullen abgefüllt und nach dem Verschließen bei 120°C 20 min sterilisiert. Jede Ampulle enthält 1 ml Lösung, entsprechend 5 mg 17S,17'S-Bissparteintetrachlorid.

Beispiel 14

Pellets

Käufliche Zuckerpellets von ca. 3 mm Durchmesser werden im rotierenden Kessel mit einer wäßrigen Lösung von 17S,17'S-Bissparteinditartrat besprüht, so daß jedes Pellet 1 mg Wirkstoff enthält, und getrocknet.

**Patentansprüche**

1. 17S,17'S-Bisspartein

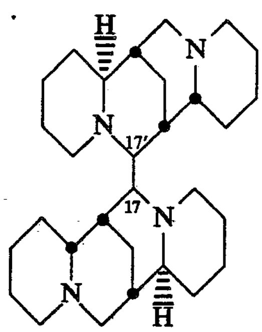

sowie seine physiologisch verträglichen Säureadditionssalze.

2. Verfahren zur Herstellung von 17S,17'S-Bisspartein, dadurch gekennzeichnet, daß man 17-Hydroxyspartein oder ein 17-Dehydrosparteinsalz mit einem Alkalimetall oder mit aktivem Magnesium in einem Lösungsmittel umsetzt und gegebenenfalls die so erhaltene Verbindung in ihre Salze mit physiologisch verträglichen Säuren überführt.

3. Verfahren nach Anspruch 2, dadurch gekennzeichnet, daß man als Dehydrosparteinsalz 17-Dehydrospartein-Perchlorat verwendet.

4. Verfahren nach einem der Ansprüche 2 oder 3, dadurch gekennzeichnet, daß man die Reaktion in aprotischen Lösungsmitteln durchführt.

5. Verfahren nach Anspruch 4, dadurch gekennzeichnet, daß man die Reaktion in Äthern, vorzugsweise in Tetrahydrofuran, Diäthyläther, Ethylenglycoldimethyläther und/oder deren Gemischen durchführt.

6. Verfahren nach einem der Ansprüche 2 bis 5, dadurch gekennzeichnet, daß man für die Umsetzung fein verteiltes Alkalimetall, vorzugsweise fein verteiltes Natrium, verwendet.

7. Verfahren nach einem der Ansprüche 2 bis 5, dadurch gekennzeichnet, daß man die Aktivierung des Magnesiums mit organischen Monohalogenverbindungen, vorzugsweise Bromveratrol und Methyljodid, vornimmt.

8. Verfahren nach einem der Ansprüche 2 bis 5, dadurch gekennzeichnet, daß man die Aktivierung des Magnesiums durch Freisetzung des Magnesiums aus einer Magnesiumhalogenverbindung, vorzugsweise Magnesiumchlorid, mit einem unedleren Metall, vorzugsweise Natrium oder Kalium, vornimmt.

9. Verfahren nach einem der Ansprüche 2 bis 5, dadurch gekennzeichnet, daß man die Aktivierung des Magnesiums durch Amalgamierung mit einer Quecksilberverbindung, vorzugsweise Quecksilber(II)-chlorid, vornimmt.

10. Verfahren nach einem der Ansprüche 2, 4 und/oder 5, dadurch gekennzeichnet, daß man die Aktivierung des Magnesiums mit einem Protonenspender vornimmt.

11. Verfahren nach Anspruch 10, dadurch gekennzeichnet, daß man als Protonenspender eine Säure verwendet.

12. Verfahren nach Anspruch 10 oder 11, dadurch gekennzeichnet, daß man die Aktivierung des Magnesiums in Gegenwart von 17-Hydroxyspartein vornimmt.

13. Verfahren nach einem der Ansprüche 10 bis 12, dadurch gekennzeichnet, daß man als Lösungsmittel ein Gemisch aus Protonenspender und aprotischem Lösungsmittel verwendet, wobei der Anteil an Protonenspender 0,5 bis 50 Vol.-%, vorzugsweise 1 bis 30 Vol.-% des Gesamtvolumens beträgt.

14. Verfahren nach einem der Ansprüche 11 bis 13, dadurch gekennzeichnet, daß man als Säure eine organische Säure, vorzugsweise Essigsäure oder Trifluoressigsäure verwendet.

15. Arzneimittel enthaltend Verbindungen gemäß Anspruch 1.

16. Verfahren zur Herstellung von Arzneimitteln mit antiarrhythmischer Wirkung, dadurch gekennzeichnet, daß man Verbindungen gemäß Anspruch 1 mit inerten, nicht toxischen, pharmazeutisch geeigneten Hilfs- und/oder Trägerstoffen mischt.

**Claims**

1. 17S,17'S-bis-sparteine

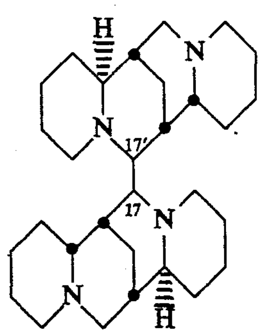

and physiologically acceptable acid addition salts thereof.

2. Process for the preparation of 17S,17'S-bissparteine, characterised in that 17-hydroxysparteine or a 17-dehydrosparteine salt is reacted with an alkali metal or with active magnesium in a solvent, and, if appropriate, the compound thus obtained is converted into its salts with physiologically acceptable acids.

3. Process according to Claim 2, characterised in that 17-dehydrosparteine perchlorate is used as the dehydrosparteine salt.

4. Process according to one of Claims 2 or 3, characterised in that the reaction is carried out in aprotic solvents.

5. Process according to Claim 4, characterised in that the reaction is carried out in ethers, preferably in tetrahydrofuran, diethyl ether, ethyleneglycol dimethyl ether and/or mixtures thereof.

6. Process according to one fo Claims 2 to 5, characterised in that a finely divided alkali metal, preferably finely divided sodium, is used for the reaction.

7. Process according to one of Claims 2 to 5, characterised in that the activation of the magnesium is carried out using organic monohalogen compounds, preferably bromoveratrol and methyl iodide.

8. Process according to one of Claims 2 to 5, characterised in that the activation of the magnesium is carried out by liberating the magnesium from a magnesium halogen compound, preferably magnesium chloride, using a non-noble metal, preferably sodium or potassium.

9. Process according to one of Claims 2 to 5, characterised in that the activation of the magnesium is carried out by amalgamation with a mercury compound, preferably mercury (II) chloride.

10. Process according to one of Claims 2, 4 and/or 5, characterised in that the activation of the magnesium is carried out with a proton donor.

11. Process according to Claim 10, characterised in that an acid is used as the proton donor.

12. Process according to Claim 10 or 11, characterised in that the activation of the magnesium is carried out in the presence of 17-hydroxysparteine.

13. Process according to one of Claims 10 to 12, characterised in that the solvent is a mixture of proton donor and aprotic solvent, the proportion of proton donor amounting to 0.5 to 50 vol.%, preferably 1 to 30 vol.-% of the total volume.

14. Process according to one of Claims 11 to 13, characterised in that the acid is an organic acid, preferably acetic acid or trifluoro acetic acid.

15. Medicaments containing compounds according to Claim 1.

16. Process for the preparation of medicaments with antiarrhythmic action, characterised in that compounds according to Claim 1 are mixed with inert, non-toxic, pharmaceutically suitable auxiliaries and/or carriers.

**Revendications**

1. 17S,17'S-bis-spartéine

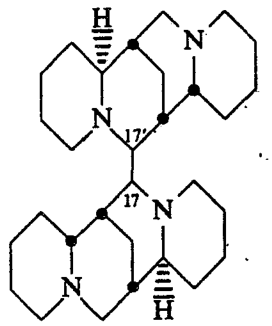

ainsi que ses sels d'addition acide compatibles d'un point de vue physiologique.

2. Procédé pour la préparation de la 17S,17'S-bisspartéine, caractérisé en ce que l'on fait réagir de la 17-hydroxyspartéine ou un sel de la 17-déhydroxpartéine sur un métal alcalin ou sur du magnésium actif dans un solvant, et, éventuellement, qu'on transforme le composé ainsi obtenu en ses sels avec des acides compatibles d'un point de vue physiologique.

3. Procédé selon la revendication 2, caractérisé en ce qu'on utilise en tant que sel de déhydrospartéine le perchlorate de 17-déhydrospartéine.

4. Procédé selon l'une des revendications 2 ou 3, caractérisé en ce qu'on réalise la réaction dans des solvants aprotiques.

5. Procédé selon la revendication 4, caractérisé en ce qu'on réalise la réaction dans des éthers, de préférence dans le tétrahydrofuranne, le diéthyléther, l'éther diméthylique de l'éthylèneglycol et/ou leurs mélanges.

6. Procédé selon l'une des revendications 2 à 5, caractérisé en ce qu'on utilise pour la réaction un métal alcalin finement divisé, de préférence du sodium finement divisé.

7. Procédé selon l'une des revendications 2 à 5, caractérisé en ce qu'on effectue l'activation du magnésium avec des composés organiques monohalogénés, de préférence le bromovératrole et l'iodure de méthyle.

8. Procédé selon l'une des revendications 2 à 5, caractérisé en ce qu'on effectue l'activation du magnésium par libération du magnésium à partir d'un composé halogéné du magnésium, de préférence le chlorure de magnésium, avec un métal commun, de préférence le sodium ou le potassium.

9. Procédé selon l'une des revendications 2 à 5, caractérisé en ce qu'on effectue l'activation du magnésium par amalgamation avec un composé du mercure, de préférence le chlorure de mercure (II).

10. Procédé selon l'une des revendications 2, 4 et/ou 5, caractérisé en ce qu'on effectue l'activation du magnésium avec un donneur de protons.

11. Procédé selon la revendication 10, caractérisé en ce qu'on utilise en tant que donneur de protons un acide.

12. Procédé selon la revendication 10 ou 11, caractérisé en ce qu'on effectue l'activation du magnésium en présence de 17-hydroxyspartéine.

13. Procédé selon l'une des revendications 10 à 12, caractérisé en ce qu'on utilise en tant que solvant un mélange de donneur de protons et d'un solvant aprotique, le pourcentage du donneur de protons étant de 0,5 à 50%-volume, de préférence de 1 à 30 %-volume du volume total.

14. Procédé selon l'une des revendications 11 à 13, caractérisé en ce qu'on utilise en tant qu'acide un acide organique, de préférence l'acide acétique ou l'acide trifluoracétique.

15. Médicament contenant des composés selon la revendication 1.

16. Procédé pour la préparation de médicaments à action anti-arythmique, caractérisé en ce qu'on mélange des composés selon la revendication 1 à des adjuvants et/ou supports inertes, non-toxiques, convenables d'un point de vue pharmaceutique.